# EUROPÄISCHE PATENTANMELDUNG

(11) **EP 3 093 643 A1**
(43) Veröffentlichungstag der Anmeldung: **16.11.2016**
(21) Anmeldenummer: 15167183.1
(22) Anmeldetag: 11.05.2015
(51) Int. Cl.: G01N 1/00, G01N 25/56, G01N 13/00

(54) **UNTERSUCHUNG VON BAUTEILEN AUF VERUNREINIGUNGEN**

(71) Anmelder: Airbus Defence and Space GmbH, 85521 Ottobrunn (DE)
(72) Erfinder: Wachinger, Georg, 85521 Ottobrunn (DE); Helwig, Andreas, 85521 Ottobrunn (DE); Meer, Thomas, 85521 Ottobrunn (DE); Geistbeck, Matthias, 85521 Ottobrunn (DE); Friedberger, Alois, 85521 Ottobrunn (DE); Heckner, Sebastian, 85521 Ottobrunn (DE)
(74) Vertreter: Isarpatent

(57) **Zusammenfassung**

Die Erfindung betrifft ein Verfahren zum Untersuchen eines Bauteils (20) auf Verunreinigung. Das Verfahren umfasst ein Aufheizen einer Probenfläche (100) des Bauteils unter einer ein Messvolumen (30) definierenden Messglocke (10); ein mindestens zweimaliges Messen einer Kontaminierung des Messvolumens mit mindestens einem Kontaminationsstoff (22); und ein Spülen des Messvolumens mit Gas.

Die Erfindung betrifft weiterhin eine Vorrichtung (1) zur Untersuchung eines Bauteils (20) auf Verunreinigung. Die Vorrichtung umfasst eine Messglocke (10), die dazu eingerichtet ist, um das Bauteil herum oder an dem Bauteil anliegend ein Messvolumen (30) auszubilden; ein Heizelement (12), das dazu eingerichtet ist, mindestens eine Probenfläche (100) des Bauteils aufzuheizen; eine Spüleinheit (14a, 14b), die dazu eingerichtet ist, das Messvolumen mit Gas zu spülen; und ein Sensorsystem (15), das dazu eingerichtet ist, eine Kontaminierung des Messvolumens mit mindestens einem Kontaminationsstoff (22) zu messen.

## Beschreibung

Die vorliegende Erfindung betrifft ein Verfahren und eine Vorrichtung zur Untersuchung von Bauteilen auf Verunreinigung.

Insbesondere Bauteile, die aus unterschiedlichen Stoffen hergestellt werden, können häufig als relativ poröses Material angesehen werden. Dies trifft beispielsweise auf Faserverbundkunststoffe zu, die Fasern und ein Matrixmaterial umfassen, wobei die Aushärtung des Materials bei der Herstellung über eine chemische Vernetzung geschieht. Unterschiedliche Stoffe, die mit derartigen Bauteilen in Berührung kommen (wie z.B. Feuchte, Reinigungsmittel, Frostschutz- oder Enteisungsmittel, Betriebsstoffe usw.) können dann gegebenenfalls in das Bauteil hineindiffundieren. Die Diffusionsgeschwindigkeit hängt einerseits von den Eigenschaften des Materials und andererseits von Parametern wie z.B. der Umgebungstemperatur, der Molekülstruktur und der Polarität des eintretenden Moleküls ab.

Umgekehrt können in ein Bauteil eingetretene Stoffe als Kontamination vom Inneren der Matrix an die Oberfläche zurückkehren und beispielsweise nachfolgende Verarbeitungsschritte wie Verbindungen mit anderen Werkstoffen, Klebungen oder Reparaturen beeinflussen.

Während der Herstellung und während des Einsatzes von Bauteilen, insbesondere von Elementen aus Faserverbundkunststoffen wie beispielsweise kohlenstofffaserverstärktem Kunststoff, ist ein Ausmaß eindiffundierter Verunreinigungen innerhalb der Struktur meist nicht bekannt.

Die Kenntnis derartiger Kontaminationen und ihrer Stärke kann jedoch für die weitere Verarbeitung wie beispielsweise für das Durchführen struktureller Klebungen (z.B. strukturell geklebter Reparaturen) bedeutsam sein, weil Kontaminationen im Werkstoff das verarbeitete Produkt beeinträchtigen können.

Beispielsweise in der zivilen Luftfahrt sind strukturelle Klebungen aus diesen Gründen nicht zulässig.

Aus der Druckschrift DE 10 2011 102 055 A1 ist eine Vorrichtung bekannt, mittels der ein Faserverbundbauteil auf das Vorhandensein einer Mehrzahl bestimmter Kontaminationsstoffe geprüft werden kann.

Die vorliegende Erfindung hat zur Aufgabe, eine verbesserte Technik bereitzustellen, mit der Bauteile auf Verunreinigungen hin untersucht werden können.

Die Aufgabe wird gelöst durch ein Verfahren gemäß Anspruch 1 und eine Vorrichtung gemäß Anspruch 13. Bevorzugte Ausführungsformen sind in den Unteransprüchen offenbart.

Ein erfindungsgemäßes Verfahren dient einer Untersuchung eines Bauteils auf Verunreinigung. Es umfasst ein Aufheizen einer Probenfläche des Bauteils unter einer ein Messvolumen definierenden Messglocke, ein mindestens zweimaliges Messen einer Kontaminierung des Messvolumens mit mindestens einem Kontaminationsstoff und ein Spülen des Messvolumens mit Gas.

Das Messen der Kontaminierung erfolgt gemäß einem erfindungsgemäßen Verfahren somit mehrere Male, mindestens zweimal. Dabei wird jeweils mindestens ein aktueller Wert gemessen, der eine Kontaminierung des Messvolumens quantifiziert, die sich gegebenenfalls aus einer durch das Aufheizen aktivierten thermischen Desorption des Kontaminationsstoffes in das Messvolumen ergeben hat; dabei kann der mindestens eine Kontaminationsstoff von der Probenfläche und/oder durch die Probenfläche aus dem Inneren des Bauteils desorbiert sein.

Eine erfindungsgemäße Vorrichtung dient einer Untersuchung eines Bauteils auf Verunreinigung und umfasst eine Messglocke, die dazu eingerichtet ist, um das Bauteil herum oder an dem Bauteil anliegend ein (vorzugsweise abgeschlossenes) Messvolumen auszubilden. Eine erfindungsgemäße Vorrichtung umfasst weiterhin ein Heizelement, das dazu eingerichtet ist, mindestens eine Probenfläche des Bauteils aufzuheizen, und eine Spüleinheit, die dazu eingerichtet ist, das Messvolumen mit Gas zu spülen; insbesondere kann die Spüleinheit einen vorzugsweise regelbaren Gasanschluss umfassen. Schließlich umfasst eine erfindungsgemäße Vorrichtung ein Sensorsystem, das dazu eingerichtet ist, eine Kontaminierung des Messvolumens mit mindestens einem Kontaminationsstoff zu messen. Je nach einer zu erfassenden Verunreinigung kann das Sensorsystem beispielsweise mindestens einen Feuchtesensor, Metalloxidsensor, Infrarot-Absorptionssensor, lonenmobilitätsspektrometer (IMS); und/oder Gas-Chromatographiesensor (der z.B. ein Ionenmobilitätsspektrometer als Detektor aufweisen kann) umfassen. Das Messen kann ein Bestimmen einer absoluten oder relativen Menge des mindestens einen Kontaminationsstoffs im Messvolumen umfassen.

Als "Bauteil" ist in dieser Schrift ein Element zu verstehen, das sich für eine weitere Verarbeitung in einem Herstellungsprozess eignet oder das bereits ein fertiges Produkt darstellt. Insbesondere umfasst der Begriff vorgeformte Elemente, die für die Herstellung eines Produktes aneinandergefügt und miteinander verbunden werden können, beispielsweise im Fahrzeugbau, insbesondere im Luftfahrzeugbau. Ein Bauteil kann mehrschichtig aufgebaut sein oder lediglich eine einzelne Schicht umfassen. Auch eine Baustoffeinheit wie beispielsweise eine Platte eines Faserverbundkunststofflaminats (z.B. eines ungehärteten Faser-Harz-Verbunds, insbesondere eine vorimprägnierte Faser-Matrix-Matte (ein sogenannter "Prepreg")) wird in dieser Schrift von dem Begriff "Bauteil" eingeschlossen. Besonders bevorzugt ist eine Ausführungsform, bei der das Bauteil ein Verbundbauteil eines Luft- oder Raumfahrzeugs ist.

Die Probenfläche kann jeweils eine Gesamtoberfläche des Bauteils oder ein lokaler Oberflächenabschnitt des Bauteils sein; an ihr erfolgt eine Untersuchung des Bauteils. Sie bildet zusammen mit der Messglocke und ggf. mit einer weiteren Fläche (beispielsweise einer Unterlage) eine Begrenzung des (um- oder anliegenden) Messvolumens. Beispielsweise kann die Messglocke in einer beliebigen Richtung auf die Probenfläche des Bauteils oder eine Unterlage gestülpt sein, oder sie kann das Bauteil ganz umschließen. Insbesondere ist der präpositionale Ausdruck "unter einer Messglocke" nicht als auf eine vertikale Ausrichtung hinweisend zu interpretieren.

Das Messvolumen kann einen beliebig geformten geometrischen Raum ausbilden (wie beispielsweise eine Kugel, eine Halbkugel, einen Zylinder, einen Kegelstumpf oder einen Quader, um nur einige zu nennen). Insbesondere ist der Begriff der das Messvolumen definierenden "Messglocke" nicht als Beschränkung der Geometrie des Messvolumens zu verstehen.

Gemäß einer bevorzugten Ausführungsform der vorliegenden Erfindung ist die Messglocke mindestens ein Teil eines tragbaren Handgeräts. Insbesondere kann sie vorzugsweise einen Durchmesser von mindestens 10 cm und/oder maximal 50 cm, bevorzugter maximal 20 cm haben, und/oder eine Masse von höchstens 5 kg, bevorzugter von höchstens 1 kg.

Besonders bevorzugt ist eine Ausführungsform, bei der die Messglocke eine Abdichtung umfasst, die das Messvolumen an einem Übergang der Messglocke zu einer Fläche abdichtet, wenn die Messglocke auf die Fläche aufgesetzt ist. Eine derartige Fläche kann beispielsweise kann eine Unterlagenoberfläche umfassen, auf der das Bauteil liegt, und/oder sie kann die Probenfläche des Bauteils umfassen.

Das Aufheizen kann von einer beliebigen Seite her erfolgen. Insbesondere kann ein verwendetes Heizelement ganz oder teilweise in dem Messvolumen oder außerhalb desselben angeordnet sein. Besonders bevorzugt ist ein Aufheizen mittels eines Energieeintrags durch ein energiedurchlässiges Fenster der Messglocke. Das Heizelement kann dabei insbesondere ein Infrarotstrahler umfassen.

Durch das Spülen des Messvolumens mit Gas wird im Messvolumen befindliches Gas ausgetauscht. Vorzugsweise wird dabei solches Gas in das Messvolumen geleitet, das vorbekannte Eigenschaften hat, insbesondere, dessen Kontaminierung mit dem mindestens einen Kontaminationsstoff einen vorbekannten Schwellwert nicht überschreitet. Gemäß einer bevorzugten Ausführungsform ist das Gas Luft, vorzugsweise synthetische Luft. Diese bietet die Vorteile, besonders kostengünstig, geruchlos, ungiftig und nicht brennbar zu sein.

Beim Spülen wird insbesondere Kontaminationsstoff aus dem Messvolumen entfernt, der gegebenenfalls zuvor von der oder durch die Probenfläche des Bauteils in das Messvolumen desorbiert ist. Das Spülen verhindert somit eine Sättigung des Messvolumens mit dem mindestens einen Kontaminationsstoff. Auf diese Weise wird eine fortgesetzte Desorption des mindestens einen Kontaminationsstoffes in das Messvolumen ermöglicht und damit ein genaues Erfassen einer Verunreinigung des Bauteils verbessert.

Die vorliegende Erfindung bietet somit insbesondere den Vorteil, dass ein thermisch aktivierter Desorptionsprozess in seinem zeitlichen Verlauf erfasst werden kann. Auf diese Weise können beispielsweise Diffusionen im Inneren des Bauteils, die als Ausgleichsentwicklungen aus der Desorption resultieren, berücksichtigt werden. Dies ermöglicht eine Analyse der Verunreinigung auch in von der Probenfläche entfernten, inneren Bereichen oder Schichten des Bauteils. Insbesondere kann auf diese Weise beispielsweise bestimmt werden, ob eine Kontamination einen Grad aufweist, der für bestimmte weitere Verarbeitungsschritte (wie beispielsweise ein strukturelles Kleben) kritisch ist. So kann erkannt werden, ob bestimmte ergänzende Maßnahmen (z.B. ein Trocknen des Bauteils) erforderlich sind. Auf diese Weise kann eine Vorbehandlung für die weiteren Verarbeitungsschritte optimiert werden, was Kosten und Arbeitsaufwand reduziert.

Gemäß einer bevorzugten Ausführungsform umfasst der Kontaminationsstoff Feuchtigkeit. Das Messen der Kontaminierung kann dabei insbesondere ein Messen einer relativen Feuchtigkeit eines im Messvolumen enthaltenen Gases (z.B. Luft) umfassen. Alternativ oder zusätzlich kann der mindestens eine Kontaminationsstoff beispielsweise Inhaltsstoffe eines oder mehrerer Reinigungs- und/oder Frostschutzmittel, Hydraulikflüssigkeit(en), Metalloxid(e), Schmierstoffe o.ä. umfassen.

Vorzugsweise erfolgt das Messen einer (aktuellen) Kontaminierung des Messvolumens mittels eines Sensorsystems. Ein derartiges Sensorsystem kann mindestens einen Feuchtesensor, mindestens einen Metalloxidsensor, mindestens einen Infrarot-Absorptionssensor, mindestens einen Gaschromatographie-Ionenmobilitätsspektrographiesensor (bzw. Gaschromatographie-Ionenmobilitätsspektrometer) und/ oder mindestens eine klassische Analyseeinheit umfassen. Beim Messen wird vorzugsweise eine absolute oder relative Menge des mindestens einen Kontaminationsstoffs im Messvolumen bestimmt.

Gemäß einer bevorzugten Ausführungsvariante der vorliegenden Erfindung wird das Spülen während des Aufheizens und/oder des (mindestens zweimaligen) Messens der Kontaminierung vorgenommen. Insbesondere kann ein Desorptionsprozess während eines fortgesetzten Spülens aktiviert und im Wege des mehrmaligen Messens der Kontaminierung des Messvolumens erfasst werden; desorbierter Kontaminationsstoff wird also in diesem Falle vorzugsweise nicht oder nur in geringem Maße im Messvolumen gesammelt. Dadurch kann direkt eine Entwicklung der Desorption über die Zeit beobachtet und zur Bestimmung einer Verunreinigung des Bauteils auch in oberflächenfernen Schichten ausgewertet werden.

Alternativ oder zusätzlich wird das Spülen während einer Spülphase vorgenommen, die zeitlich zwischen einer ersten und einer zweiten Aktivierungsphase (also nach der ersten und vor der zweiten Aktivierungsphase) liegt. Die erste und die zweite Aktivierungsphase umfassen dabei jeweils mindestens ein einmaliges Messen der (dann jeweils aktuellen) Kontaminierung des Messvolumens mit dem Kontaminationsstoff, wobei das Messvolumen während der ersten und der zweiten Aktivierungsphase vorzugsweise abgeschlossen ist. Vorzugsweise erfolgt das Aufheizen der Probenfläche mindestens teilweise während der ersten und der zweiten Aktivierungsphase, in denen dadurch jeweils - sofern eine Verunreinigung des Bauteils vorliegt - eine Desorption des mindestens einen Kontaminationsstoffes thermisch aktiviert werden kann.

Auf diese Weise kann desorbierter Kontaminationsstoff zeitweise im Messvolumen gesammelt und die fortschreitende Desorption während der einzelnen Aktivierungsphasen verfolgt werden. Durch das Spülen zwischen den beiden Aktivierungsphasen kann dafür gesorgt werden, dass die Ausgangsbedingungen in beiden Phasen übereinstimmen; insbesondere kann eine Verminderung der Desorption infolge eines Sättigungsprozesses des Messvolumens mit dem desorbierten Kontaminationsstoff verhindert werden. Ein Messen der fortschreitenden Kontaminierung im abgeschlossenen Messvolumen ermöglicht ein präzises Erfassen der Kontaminierung, bei dem insbesondere bei langsam ablaufenden Desorptionsprozessen Messwerte unterhalb einer Erfassungsschwelle vermieden werden können.

Während der Spülphase kann das Messen der Kontaminierung fortgesetzt oder wiederholt werden. Die Spülphase kann somit ein- oder mehrmaliges Messen einer (jeweils aktuellen) Kontaminierung des Messvolumens mit dem Kontaminationsstoff während des Spülens umfassen. Auf diese Weise kann das Gas im Messvolumen auch während des Spülens kontrolliert werden.

Insbesondere kann eine Dauer der Spülphase abhängig von einer Kontaminierung des Gases gesteuert sein. Dazu kann das Verfahren ein Vergleichen einer aktuell gemessenen Kontaminierung mit einem Vergleichswert und ein Feststellen umfassen, dass die gemessene Kontaminierung kleiner oder gleich einem Vergleichswert ist. Das Spülen kann dann insbesondere durch ein Schließen eines Gasanschlusses in das Messvolumen erfolgen, vorzugsweise zudem durch ein Schließen eines Gasauslasses des Messvolumens.

Auf diese Weise kann ohne Zeitverlust wegen einer ggf. unnötig langen Fortsetzung des Spülens erreicht werden, dass das Gas im Messvolumen zu Beginn der zweiten Aktivierungsphase eine vorbestimmte Qualität hat. Dadurch können sowohl geeignete Desorptionsvoraussetzungen geschaffen, als auch in einer Auswertung der gemessenen Kontaminierung eine Verunreinigung des Bauteils präzise quantifiziert werden.

Das Spülen wird vorzugsweise automatisch gesteuert. Insbesondere umfasst eine verwendete Spüleinheit vorzugsweise einen Regler, der Beginn und Ende des Spülens, eine durchgeleitete Gasmenge und/oder einen Spüldruck automatisch steuert.

Gemäß einer bevorzugten Ausführungsform eines erfindungsgemäßen Verfahrens erfolgt je ein mindestens einmaliges Messen der Kontaminierung des Messvolumens vor und nach einer Ausgleichsphase einer vorbestimmten Dauer; die vorbestimmte Dauer kann beispielsweise mehrere Stunden oder sogar Tage umfassen. Während der Ausgleichsphase wird die Probenfläche des Bauteils (ggf. in einem jeweils untersuchten Abschnitt) auf eine vorbestimmte Temperatur temperiert, die unterhalb einer Desorptionstemperatur liegt, bei der also keine thermische Aktivierung einer Desorption des mindestens einen Kontaminationsstoffes erfolgt. Die Temperaturen können dabei insbesondere in Abhängigkeit von der jeweiligen Kontamination und/oder dem Werkstoff gewählt werden oder worden sein. Beispielsweise kann die Ausgleichsphase ein Einlagern des Bauteils in einer Wärmekammer oder einem Ofen bei einer Temperatur umfassen, die (insbesondere im Falle von Feuchtigkeit als Kontamination) beispielsweise in einem Bereich von 20° - 45°C, bevorzugter 35°C - 40°C liegen kann.

Nach der Ausgleichsphase erfolgt vorzugsweise ein erneutes Erhitzen der Probenfläche des Bauteils. Eine oder mehrere der Messungen nach der Ausgleichsphase können vorzugsweise von einer weiteren Spülphase analog zu den oben geschilderten unterbrochen sein. Insbesondere kann das Messen nach der Ausgleichsphase in einer dritten und vierten Aktivierungsphase erfolgen, die analog zu einer ersten und zweiten Aktivierungsphase ablaufen und voneinander durch eine entsprechende Spülphase getrennt sein können.

Infolge einer Desorption von der Probenfläche her kann eine Verteilung einer Verunreinigung im Innern des Bauteils mit zunehmendem Abstand von der Oberfläche (beispielsweise in verschiedenen Lagen eines mehrschichtigen Bauteils) stark variieren. Die Ausgleichsphase kann dabei dafür sorgen, dass entstandene Konzentrationsunterschiede durch Diffusion im Innern des Bauteils ausgeglichen und so ein Gleichgewicht hinsichtlich der Verteilung wiederhergestellt wird. Insbesondere kann dadurch erreicht werden, dass weiter im Innern des Bauteils liegende Kontaminationsteilchen zur Oberfläche (insbesondere zur Probenfläche) diffundieren und so einer Desorption durch die Probenfläche zugänglich werden.

Gemäß einer bevorzugten Ausführungsform umfasst ein erfindungsgemäßes Verfahren ein Quantifizieren einer Verunreinigung auf Grundlage der (gegebenenfalls z.B. während der Aktivierungs- und/oder Spülphasen) gemessenen Kontaminierungen des Messvolumens; eine erfindungsgemäße Vorrichtung ist analog vorzugsweise dazu eingerichtet, eine derartige Quantifizierung vorzunehmen. Die Quantifizierung kann einen inneren Bereich des Bauteils (insbesondere einen inneren Bereich, der nach außen hin von der Probenfläche abgeschlossen wird) und/oder die Probenfläche des Bauteils und/oder das gesamte Bauteil betreffen.

Mittels so erhaltener Mengenangaben kann beispielsweise eine Eignung des Bauteils zu weiteren Verarbeitungsschritten (z.B. Kleben) und/ oder ein Erfordernis des Ergreifens von Gegenmaßnahmen bestimmt werden. Beispielsweise kann im Falle, dass der mindestens eine Kontaminationsstoff Wasser umfasst, auf diese Weise ermittelt werden, ob ein Trocknen erforderlich ist und ggf., wie lange und/oder bei welcher Temperatur ein derartiger Trocknungsprozess erfolgen sollte. Alternativ oder zusätzlich kann/ können für die jeweiligen Kontamination eine oder mehrere Toleranzgrenze/n bestimmt werden. Liegen die bestimmten quantitativen Kontaminationen unterhalb der Toleranzgrenze/n, kann auf nachfolgende Trocknungsvorgänge verzichtet werden.

Das Quantifizieren kann insbesondere ein Addieren von Kontaminierungen (also von Messwerten, die die jeweiligen Kontaminierungen wiedergeben) umfassen, wie beispielsweise ein Addieren von in einer zweiten Aktivierungsphase (wie oben beschrieben) ermittelten Kontaminierungen zu einer (z.B. maximalen) Kontaminierung, die in einer vorherigen, ersten Aktivierungsphase gemessen wurde. Auf diese Weise kann die Menge des bis zum jeweiligen Zeitpunkt insgesamt in das Messvolumen desorbierten Kontaminationsstoffes ermittelt werden, von dem sich während der zweiten Aktivierungsphase infolge des Spülens ein Teil nicht mehr im Messvolumen befindet.

Besonders bevorzugt ist eine Ausführungsform, bei der das Quantifizieren unter Berücksichtigung der jeweiligen Messzeitpunkte erfolgt, zu denen die jeweiligen Kontaminierungen erfasst wurden.

Alternativ oder zusätzlich kann das Quantifizieren beispielsweise eine Bestimmung und Analyse einer Interpolationsfunktion zu den gemessenen Kontaminierungen in Abhängigkeit von der Zeit umfassen; eine derartige Analyse kann ein Differenzieren und/oder Integrieren und/oder eine Bestimmung von Extremwerten, insbesondere Maxima der Interpolationsfunktion umfassen.

Das Quantifizieren kann ein Vergleichen mindestens einer gemessenen Kontaminierung, vorzugsweise eines Verlaufs einer mehrere Kontaminierungsmessungen umfassenden Messreihe mit einem oder mehreren in einer Datenbank gespeicherten und/oder mittels Simulation bestimmten Wert(en) umfassen.

Besonders bevorzugt ist eine Ausführungsform, bei der das Quantifizieren ein Erstellen eines Kontaminationsprofils umfasst, das eine Verunreinigungsverteilung in einem Querschnitt des Bauteils wiedergibt.

Eine bevorzugte Ausführungsform einer erfindungsgemäßen Vorrichtung umfasst eine Rechnereinheit, die dazu eingerichtet ist, auf Grundlage gemessener Kontaminierungen des Messvolumens eine Verunreinigung im Innern des Bauteils und/oder auf einer Oberfläche des Bauteils zu quantifizieren (beispielsweise in einer der oben angegebenen Weisen) und/oder eine Verunreinigungsverteilung im Innern des Bauteils zu bestimmen.

Gemäß einer bevorzugten Ausführungsvariante ist die Probenfläche des Bauteils ein erster Probenflächenabschnitt und umfasst ein erfindungsgemäßes Verfahren ein Aufheizen eines zweiten Probenflächenabschnitts des Bauteils unter der (umpositionierten) Messglocke oder unter einer weiteren Messglocke; in beiden Fällen definiert die jeweilige (d.h. die umpositionierte oder die weitere) Messglocke ein weiteres Messvolumen, das an den zweiten Probenflächenabschnitt des Bauteils angrenzt; es versteht sich, dass die oben dargelegten Feststellungen über die Bezeichnungen "Messglocke" und "unter der Messglocke" auch in Bezug auf den zweiten Probenflächenabschnitt gelten. Das Verfahren kann ferner ein mindestens zweimaliges Messen einer Kontaminierung des weiteren Messvolumens mit dem mindestens einen Kontaminationsstoff und ein Spülen des weiteren Messvolumens mit Gas umfassen.

Eine Messglocke einer bevorzugten Ausführungsform der vorliegenden Erfindung ist analog vorzugsweise dazu eingerichtet, nacheinander auf verschiedene Abschnitte des Bauteils und/oder verschiedener Bauteile aufgesetzt zu werden, um mit dem jeweils anliegenden Abschnitt jeweils ein (vorzugsweise abgeschlossenes) Messvolumen auszubilden.

Insbesondere kann das Bauteil somit stichprobenartig an mindestens einem weiteren Probenflächenabschnitt gemäß einem oder mehreren (ggf. unterschiedlichen) der hier beschriebenen Verfahren untersucht. Die so erhaltenen, gegebenenfalls jeweils lokale Verunreinigungen widerspiegelnden Ergebnisse können dann zur Bestimmung einer Verunreinigung des gesamten Bauteils (oder eines mehrere untersuchte Probenflächenabschnitte umfassenden, größeren Abschnitts eines Bauteils) quantifiziert werden, und/oder es kann eine Verunreinigungsverteilung im Bauteil (bzw. dem größeren Abschnitt) bestimmt werden. Auf diese Weise können (ggf. unter Heranziehung statistischer Verfahren) auch große Bauteile auf Verunreinigungen hin untersucht werden.

Gemäß einer bevorzugten Ausführungsform der vorliegenden Erfindung enthält das Bauteil einen Faserverbundkunststoff oder besteht das Bauteil aus einem Faserverbundkunststoff. Insbesondere kann das Bauteil ein Laminat und/oder ein Prepreg umfassen. Es kann beispielsweise einen mehrschichtigen kohlenstofffaserverstärkten Kunststoff (CFK) enthalten. Derartige Materialien können besonders gut mit einem erfindungsgemäßen Verfahren bzw. einer erfindungsgemäßen Vorrichtung auf Verunreinigungen untersucht werden. Insbesondere eignet sich dabei ein zyklisches Vorgehen, das eine Ausgleichsphase wie geschildert umfasst und so eine allmähliche Diffusion des mindestens einen Kontaminationsstoffs aus tieferliegenden Schichten des Bauteils berücksichtigt.

Im Folgenden werden bevorzugte Ausführungsbeispiele der Erfindung anhand von Zeichnungen näher erläutert. Es versteht sich, dass einzelne Elemente und Komponenten auch anders kombiniert werden können als dargestellt.

Es zeigen schematisch:
- Figur 1: eine Vorrichtung gemäß einer beispielhaften Ausführungsform der vorliegenden Erfindung;
- Figur 2: eine beispielhafte Messreihe als ein Ergebnis einer Anwendung eines erfindungsgemäßen Verfahrens
- Figur 3: eine beispielhaftes Feuchteprofil eines Bauteils nach einer mehreren Verfahrenszyklen.

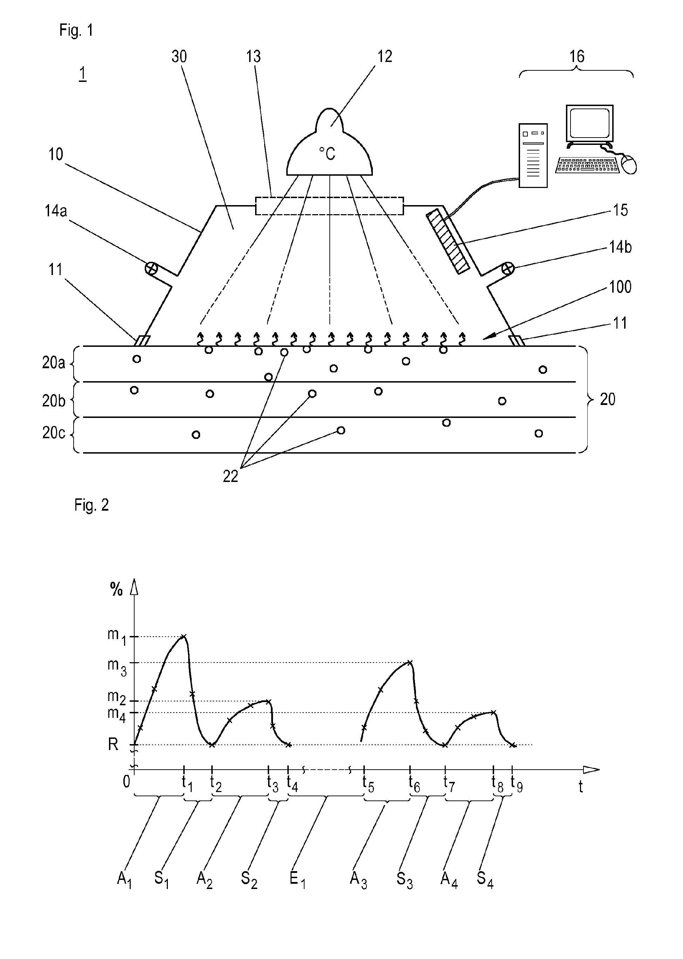

In Figur 1 ist (nicht maßstabsgetreu) eine Anordnung schematisch dargestellt, die eine Ausführungsform einer erfindungsgemäßen Vorrichtung 1 umfasst.

Die Vorrichtung umfasst eine Messglocke 10, die an einem Bauteil 20 anliegend ein Messvolumen 30 ausbildet. Insbesondere wird das Messvolumen 30 von der Messglocke und einer Probenfläche 100, die Teil einer Oberfläche des Bauteils 20 ist, eingeschlossen.

Die Messglocke 10 weist an ihrem Rand eine Abdichtung 11 auf, die das Messvolumen an einem Übergang zwischen der Messglocke 10 und der Probenfläche 100 abdichtet.

Das Bauteil 20 umfasst im dargestellten Beispiel einen schichtweisen Aufbau (z. B. Laminat) mit mehreren Lagen 21a, 21b und 21c.

Die Lagen können jeweils beispielsweise ein Faser-Matrix-Verbundmaterial umfassen. Das Bauteil umfasst weiterhin in seinem Innern in verschiedenen einen Kontaminationsstoff 22, der in verschiedene Schichten des Bauteilinneren (also in eine Bauteiltiefe) eingedrungen ist.

Die Vorrichtung 1 umfasst weiterhin ein Heizelement 12, das dazu eingerichtet ist, die Probenfläche 100 aufzuheizen. Im dargestellten Ausführungsbeispiel ist das Heizelement außerhalb der Messglocke 10 angeordnet, und sie erwärmt die Probenfläche durch ein in der Messglocke 10 befindliches energiedurchlässiges Fenster 13.

Eine Spüleinheit 14a, 14b umfasst einen vorzugsweise regelbaren Gasanschluss 14a und einen Gasauslass 14b und ist dazu eingerichtet, ein vorbestimmtes Gas (beispielsweise synthetische Luft) durch das Messvolumen hindurchzuleiten und dabei zusammen mit dem Gas Kontaminationsstoff aus dem Messvolumen 30 hinauszuspülen.

Die Vorrichtung 1 umfasst ferner ein Sensorsystem 15 (mit einem oder mehreren Sensoren), das dazu eingerichtet ist, eine Kontaminierung des Messvolumens 30 mit (mindestens) dem Kontaminationsstoff 22 zu messen. Schließlich umfasst die Vorrichtung 1 eine Rechnereinheit 16, die an das Sensorsystem 15 angeschlossen und dazu eingerichtet ist, auf Grundlage vom Sensorsystem 15 gemessener Kontaminierungen eine Verunreinigung im Innern des Bauteils 20 und/oder auf der Probenfläche zu quantifizieren und/oder eine Verunreinigungsverteilung im Innern des Bauteils 20 zu bestimmen.

In Verwendung der Vorrichtung wird nun die Probenfläche 100 mit Hilfe des Heizelements 12 erhitzt, was eine Desorption des Kontaminationsstoffes 22 aus einer oberflächennahen Schicht 20a in das Messvolumen 30 bewirkt, wie durch die Pfeile dargestellt. Das Sensorsystem 15 misst die daraus resultierende Kontaminierung des Messvolumens, vorzugsweise mehrere Male, und leitet die jeweils gemessenen Werte an die Recheneinheit 16 weiter.

Nach einer vorbestimmten Zeit (z. B. 5 Minuten) wird das Messvolumen mit Hilfe der Spüleinheit 14a, 14b durch Durchleiten von Gas gespült. Dabei wird das Heizen vorzugsweise ausgesetzt. Während des Spülens kann weiterhin ein Messen einer (jeweils aktuellen) Kontaminierung des Messvolumens 30 mit dem mindestens einen Kontaminationsstoff 22 erfolgen. Nach Feststellung, dass ein vorgegebener Vergleichswert für eine Kontaminierung des Messvolumens unterschritten wurde, oder nach Ablauf einer vorbestimmten Zeit wird das Spülen beendet. Es kann nun ein erneutes Messen einer Kontaminierung des Messvolumens mit dem Kontaminationsstoff 22 erfolgen, nach der das Bauteil in einer Ausgleichsphase für eine vorbestimmte Dauer auf eine Temperatur unterhalb Desorptionstemperatur temperiert wird, ehe die Probenfläche 100 erneut erhitzt und eine Kontaminierung des Messvolumens 30 gemessen wird.

Alternativ kann die Ausgleichsphase gleich an das Spülen angeschlossen werden, ohne dass zuvor ein erneutes Messen einer Kontaminierung des Messvolumens erfolgt. Während der Ausgleichsphase diffundieren Teile des Kontaminationsstoffs 22 aus einer tieferen (oberflächenferneren) Schicht 21 b des Bauteils 20 in eine oberflächennähere Schicht 20a. Von dort aus kann der Kontaminationsstoff infolge der thermischen Aktivierung nach der Ausgleichsphase in das Messvolumen desorbieren und dort gemessen werden. Eine Auswertung der Kontaminierung unter Berücksichtigung der Zeit der jeweiligen Messung erlaubt eine Bestimmung einer Verunreinigungsverteilung im Innern des Bauteils 20.

Figur 2 zeigt eine Messreihe als Ergebnis einer Durchführung eines erfindungsgemäßen Verfahrens in einer Ausführungsform, die als Funktion über die Zeit dargestellt ist. Zwischenwerte wurden in der Darstellung interpoliert. Der Kontaminationsstoff kann beispielsweise Wasser und die gemessene Kontaminierung als relative Luftfeuchtigkeit gemessen sein.

Die Messreihe spiegelt eine erste bis vierte Aktivierungsphase A₁, A₂, A₃, A₄ wider, während deren jeweils eine Desorption von Probenfläche thermisch aktiviert wurde. Wie an der graphischen Darstellung der Messreihe erkennbar, stieg die Kontaminierung des Messvolumens in den verschiedenen Aktivierungsphasen auf die jeweiligen Maximalwerte m₁, m₂, m₃ und m₄ an.

Die jeweilige Dauer der einzelnen Phasen kann geeignet gewählt sein; beispielsweise können für die erste und die dritte Aktivierungsphase A₁ bzw. A₃ jeweils 3 bis 5 Minuten gewählt sein und für die zweite und die vierte Aktivierungsphase A₂ bzw. A₄ jeweils 8 bis 12 Minuten. Gemäß einem Ausführungsbeispiel dauern die zweite und die vierte Aktivierungsphase jeweils länger als die erste bzw. die dritte Aktivierungsphasen.

Nach den einzelnen Aktivierungsphasen erfolgten Spülphasen S₁, S₂, S₃ und S₄, während deren das Messen der Kontaminierung fortgesetzt wurde. Die Spülphasen wurden jeweils beendet, wenn die Kontaminierung im Messvolumen auf einen unteren Schrankenwert R als Vergleichswert abgefallen war.

Zwischen der Spülphase S₂ und der dritten Aktivierungsphase A₃ erfolgte eine Ausgleichsphase E₁, während der das Bauteil auf eine vorbestimmte Temperatur temperiert wurde, die unterhalb einer Desorptionstemperatur liegt. Gemäß einem speziellen Beispiel kann die Kontamination eine Feuchtigkeit betreffen, die Temperatur kann bei etwa 38°C gelegen haben, und die Ausgleichsphase E₁ kann 19 Stunden oder mehr gedauert haben; es versteht sich, dass andere Kontaminationen, Temperaturen und/oder Dauern vorgesehen sein können. Im in Figur 2 gezeigten Beispiel konnte im Innern des Bauteils vorhandener Kontaminationsstoff während der Ausgleichsphase in eine oberflächennähere Schicht diffundieren, um dann in der dritten Aktivierungsphase aus der Probenfläche heraus zu desorbieren. Mit dieser Diffusion erklärt sich, dass die in der dritten Aktivierungsphase gemessene Kontamination m₃ des Messvolumens im vorliegenden Fall größer ist als die zuvor während der zweiten Aktivierungsphase A₂ gemessene Kontaminierung m₂. Insbesondere ergibt die Messreihe, dass in dem betrachteten Beispiel vor Beginn der Messungen Kontaminationsstoff auch in tieferen (oberflächenferneren) Schichten vorhanden war, und die entsprechende Menge kann anhand der Messreihe bestimmt werden.

An die Spülphase S₄ kann sich gemäß einer bevorzugten Ausführungsform mindestens eine weitere Ausgleichsphase anschließen, die die gleiche oder eine andere vorbestimmte Dauer wie/als die erste Ausgleichsphase haben kann und nach der mindestens eine weitere Aktivierungsphase A₅ folgen kann.

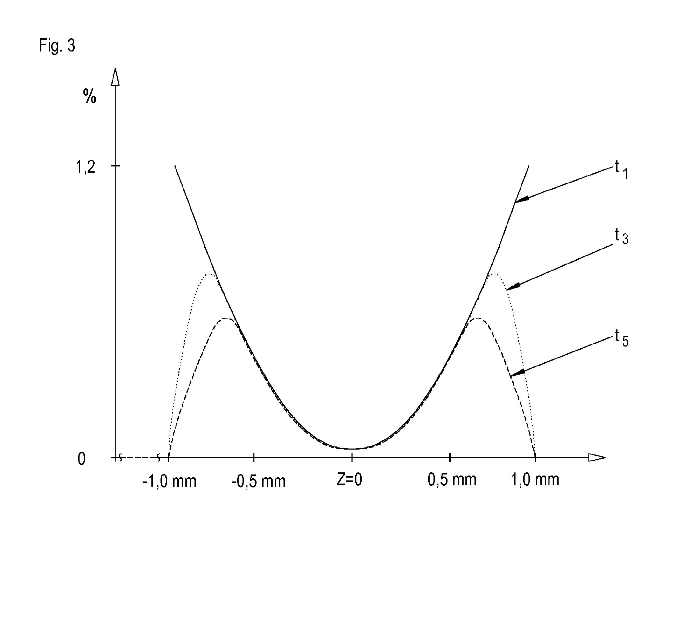

Figur 3 zeigt beispielhaft drei Feuchteprofile eines 2 mm dicken Bauteils nach jeweils einer anderen Aktivierungsphase; das Zentrum des Bauteils ist dabei vorliegend im Nullpunkt dargestellt; in den inneren Lagen weist das dargestellte Bauteil keine Kontamination auf.

Die Veränderung des Feuchteprofils entspricht jeweils dem ausgegasten Volumen der Verunreinigung und korreliert direkt mit den gemessenen Kontaminierungen. Vergleicht man also die gemessenen Kontaminierungen mit den ermittelten Differenzprofilen, kann man direkt auf die quantitative Verunreinigung und/oder auf die Kontaminationsverteilung in Dickenrichtung schließen.

### Bezugszeichen

- 1: Vorrichtung
- 10: Messglocke
- 11: Abdichtung
- 12: Heizelement
- 13: Fenster
- 14a, 14b: Spüleinheit
- 15: Sensorsystem
- 16: Recheneinheit
- 20: Bauteil
- 21a, 21b, 21c: Bauteilschicht
- 22: Kontaminationsstoff
- 30: Messvolumen
- 100: Probenfläche

- A₁, A₂, A₃, A₄, A₅: Aktivierungsphasen
- S₁, S₂, S₃, S₄: Spülphasen
- E₁: Ausgleichsphase
- R: Vergleichswert

## Patentansprüche

1. Verfahren zum Untersuchen eines Bauteils (20) auf Verunreinigung, wobei das Verfahren umfasst:
ein Aufheizen einer Probenfläche (100) des Bauteils unter einer ein Messvolumen (30) definierenden Messglocke (10);
ein mindestens zweimaliges Messen einer Kontaminierung des Messvolumens mit mindestens einem Kontaminationsstoff (22); und
ein Spülen des Messvolumens mit Gas.

2. Verfahren gemäß Anspruch 1, bei dem das Spülen während des Aufheizens und/oder des mindestens zweimaligen Messens erfolgt.

3. Verfahren gemäß Anspruch 1, wobei das Spülen während einer Spülphase (S₁, S₂, S₃, S₄) geschieht, die zwischen einer ersten und einer zweiten Aktivierungsphase (A₁, A₂, A₃, A₄) liegt, und wobei die erste und die zweite Aktivierungsphase jeweils mindestens ein einmaliges Messen der Kontaminierung des Messvolumens mit dem Kontaminationsstoff umfassen.

4. Verfahren gemäß Anspruch 3, wobei die Spülphase weiterhin ein ein- oder mehrmaliges Messen einer Kontaminierung des Messvolumens mit dem Kontaminationsstoff während des Spülens umfasst.

5. Verfahren gemäß Anspruch 4, bei dem die Spülphase weiterhin umfasst:
ein Feststellen, dass die gemessene Kontaminierung kleiner oder gleich einem Vergleichswert (R) ist; und
ein Beenden des Spülens durch Schließen eines Gasanschlusses (14a) und eines Gasauslasses (14b).

6. Verfahren gemäß einem der vorhergehenden Ansprüche, wobei mindestens ein Messen der Kontaminierung vor und ein Messen der Kontaminierung nach einer Ausgleichsphase (E₁) erfolgt, die ein Temperieren der Probenfläche (100) für eine vorbestimmte Zeit auf eine vorbestimmte Temperatur unterhalb einer Desorptionstemperatur umfasst.

7. Verfahren gemäß einem der vorhergehenden Ansprüche, das zudem ein Quantifizieren einer Verunreinigung im Innern des Bauteils (20) und/oder an der Probenfläche (100) des Bauteils auf Grundlage der gemessenen Kontaminierungen des Messvolumens umfasst.

8. Verfahren gemäß Anspruch 7, bei dem das Quantifizieren ein Bestimmen einer Verunreinigungsverteilung im Innern des Bauteils umfasst; und/oder bei dem das Quantifizieren auf Grundlage der gemessenen Kontaminierungen eine Bestimmung und Analyse einer Interpolationsfunktion zu den gemessenen Kontaminierungen umfasst.

9. Verfahren gemäß einem der vorhergehenden Ansprüche, bei dem die Probenfläche (100) ein erster Probenflächenabschnitt des Bauteils ist, und wobei das Verfahren weiterhin umfasst:
ein Aufheizen eines zweiten Probenflächenabschnitts des Bauteils unter der Messglocke (10) oder unter einer weiteren Messglocke, die jeweils ein weiteres Messvolumen definiert;
ein mindestens zweimaliges Messen einer Kontaminierung des weiteren Messvolumens mit dem mindestens einen Kontaminationsstoff (22); und
ein Spülen des weiteren Messvolumens mit Gas.

10. Verfahren gemäß einem der vorhergehenden Ansprüche, bei dem das Bauteil (20) einen Faserverbundkunststoff enthält oder aus einem Faserverbundkunststoff besteht.

11. Verfahren gemäß einem der vorhergehenden Ansprüche, bei dem der mindestens eine Kontaminationsstoff (22) Feuchtigkeit umfasst.

12. Verfahren gemäß einem der vorhergehenden Ansprüche, bei dem das Messen einer Kontaminierung des Messvolumens (30) mittels eines Sensorsystems (15) erfolgt, das umfasst: mindestens einen Feuchtesensor, mindestens einen Metalloxidsensor, mindestens einen Infrarot-Absorptionssensor und/oder mindestens einen Gaschromatographie-Ionenmobilitätsspektrographiesensor.

13. Vorrichtung (1) zur Untersuchung eines Bauteils (20) auf Verunreinigung, die umfasst:
eine Messglocke (10), die dazu eingerichtet ist, um das Bauteil herum oder an dem Bauteil anliegend ein Messvolumen (30) auszubilden;
ein Heizelement (12), das dazu eingerichtet ist, mindestens eine Probenfläche (100) des Bauteils aufzuheizen;
eine Spüleinheit (14a, 14b), die dazu eingerichtet ist, das Messvolumen mit Gas zu spülen; und
ein Sensorsystem (15), das dazu eingerichtet ist, eine Kontaminierung des Messvolumens mit mindestens einem Kontaminationsstoff (22) zu messen.

14. Vorrichtung gemäß Anspruch 13, die eine Rechnereinheit (16) umfasst, die dazu eingerichtet ist, auf Grundlage gemessener Kontaminierungen des Messvolumens eine Verunreinigung im Innern des Bauteils und/oder auf einer Oberfläche des Bauteils zu quantifizieren und/oder eine Verunreinigungsverteilung im Innern des Bauteils zu bestimmen.

15. Vorrichtung gemäß Anspruch 13 oder 14, wobei die Messglocke (10) dazu eingerichtet ist, nacheinander auf verschiedene Abschnitte des Bauteils und/oder verschiedener Bauteile aufgesetzt zu werden, um mit dem jeweils anliegenden Abschnitt jeweils ein Messvolumen auszubilden.
